# EUROPEAN PATENT APPLICATION

(11) **EP 4 585 677 A1**
(43) Date of publication of application: **16.07.2025**
(21) Application number: 23880062.7
(22) Date of filing: 22.09.2023
(51) Int. Cl.: C12N 1/14, A01N 63/30, A01P 3/00, A01P 7/02, A01G 7/06, C12R 1/645

(54) **NOVEL BEAUVERIA BASSIANA 331R STRAIN WITH INSECTICIDAL AND BACTERICIDAL ACTIVITY AND USE THEREOF**

(30) Priority: 21.10.2022 KR 20220136800
(71) Applicant: Solvum Co., Ltd., Daejeon 34014 (KR)
(72) Inventor: WOO, Soo Dong, Cheongju-si Chungcheongbuk-do 28617 (KR); LEE, Jin Yong, Cheongju-si Chungcheongbuk-do 28390 (KR); WOO, Ra Mi, Cheongju-si Chungcheongbuk-do 28645 (KR)
(74) Representative: Isarpatent
(86) International application number: PCT/KR2023/014513
(87) International publication number: WO 2024/085483

(57) **Abstract**

The present disclosure relates to a novel *Beauveria bassiana* 331R strain with insecticidal and bactericidal activity and use thereof and, in particular, to a novel *Beauveria bassiana* 331R strain (Accession No.: KACC83066BP) having control activity against both plant pests and phytopathogens, a composition for simultaneous control of pests and plant diseases, comprising the *Beauveria bassiana* 331R strain as an active ingredient, and a simultaneous control method for pests and plant diseases using the *Beauveria bassiana* 331R strain. The *Beauveria bassiana* 331R strain according to the present disclosure is a pest control source that is safe for humans and animals and the environmental ecosystem and can simultaneously control pests harmful to crops and various phytopathogens, and thus can be effectively used as a new biological agent.

## Description

### Technical Field

The present disclosure relates to a novel entomopathogenic fungus strain, *Beauveria bassiana* 331R, which exhibits pesticidal and fungicidal activities against plant pests and phytopathogenic microorganisms, as well as a use thereof.

### Background Art

Various crop protection agents have been used to control plant pests and plant diseases, which are considered the major obstacles to plant growth. Research for developing more effective crop protection agents is being actively conducted worldwide. Crop protection agents are essential not only for economic purposes, such as food production, but also for landscaping, and their associated economic costs are considerably high. Until now, most crop protection agents have been developed using chemical substances. Consequently, concerns about the adverse effects of these chemical-based agents on human health and the environment, including ecosystem destruction, have been raised. To address the side effects of these crop protection agents, various ecofriendly pest control methods have been developed, among which microbial insecticides utilizing entomopathogenic microorganisms have gained significant attention.

Microorganisms that can serve as microbial insecticides include various types of microorganisms such as bacteria, viruses, and fungi, with entomopathogenic fungi having the longest history of use. Entomopathogenic fungi cause diseases in insects and induce mortality by invading the insect host and producing various extracellular enzymes, toxins, and secondary metabolites during their proliferation. These compounds help the fungi overcome the host insect's immune response, ultimately leading to the host's death, and also facilitate competition against other microorganisms.

Effective control of pests and plant diseases that damage crops necessitates the use of separate crop protection agents for each specific target. However, the development of crop protection agents capable of simultaneously controlling both plant pests and phytopathogens remains insufficient.

### Disclosure of Invention

### Technical Problem

Leading to the present disclosure, intensive and thorough research conducted by the present inventors, who successfully have isolated and identified a novel entomopathogenic fungus strain, *Beauveria bassiana* 331R, from *Tetranychus urticae,* resulted in the finding that the strain exhibits pesticidal effects against *Tetranychus urticae, Myzus persicae,* and *Aphis gossypii,* as well as potent fungicidal activity against various phytopathogenic microorganisms, thus finding applications as a novel crop protection agent for the simultaneous control of both pests and phytopathogens.

Therefore, an aspect of the present disclosure is to provide a *Beauveria bassiana* 331R strain (Accession No.: KACC83066BP) exhibiting pest and phytopathogen control activity.

Another aspect of the present disclosure is to provide a composition comprising the strain, spores thereof, a culture thereof, a concentrate of the culture, or a dried form of the culture as an active ingredient for the simultaneous control of pests and phytopathogens.

Yet another aspect of the present disclosure is to provide a method for simultaneously controlling pests and phytopathogens, the method comprising a step of applying the composition for the simultaneous control of pests and phytopathogens of the present disclosure to areas where pests and phytopathogens proliferate.

### Solution to Problem

To achieve the above goals, the present disclosure provides a *Beauveria bassiana* 331R strain (Accession No.: KACC83066BP) exhibiting biocidal activity against both pests and phytopathogens.

In an embodiment of the present disclosure, the pests may include *Tetranychus urticae, Myzus persicae,* or *Aphis gossypii.*

In an embodiment of the present disclosure, the biocidal activity against phytopathogens refers to fungicidal activity against fungi that cause plant diseases.

In an embodiment of the present disclosure, the fungi may include *Botrytis cinerea* T3-4, *Colletotrichum acutatum* 15AS32, or *Colletotrichum fructicola* CGF160401.

Additionally, the present disclosure provides a composition comprising *Beauveria bassiana* 331R (Accession No.: KACC83066BP), spores thereof, a culture thereof, a concentrate of the culture, or a dried form of the culture as an active ingredient for the simultaneous control of pests and plant diseases.

In an embodiment of the present disclosure, the pests may include *Tetranychus urticae, Myzus persicae,* or *Aphis gossypii.*

In an embodiment of the present disclosure, the plant diseases may be caused by *Botrytis cinerea* T3-4, *Colletotrichum acutatum* 15AS32, or *Colletotrichum fructicola* CGF160401.

Furthermore, the present disclosure provides a method for simultaneously controlling pests and plant diseases, comprising a step of applying the composition for the simultaneous control of pests and plant diseases of the present disclosure to areas where pests and phytopathogens proliferate.

In an embodiment of the present disclosure, the target area may include soil or crops.

In an embodiment of the present disclosure, the crops may include eggplant, chili pepper, tomato, strawberry, Chinese cabbage, pear, apple, cucumber, rose, cherry, pittosporum, rugosa rose, Japanese apricot, tree of heaven, citrus, potato, sweet potato, tobacco, cotton, spindle tree, oleander, beautyberry, goji berry, paulownia, apricot, plum, or persimmon tree.

### Advantageous Effects of Invention

The *Beauveria bassiana* 331R strain of the present disclosure exhibits excellent pesticidal effects against *Tetranychus urticae, Myzus persicae,* and *Aphis gossypii,* while simultaneously exhibiting fungicidal activity against various phytopathogens. Furthermore, the *Beauveria bassiana* 331R strain is a safe pest control agent for humans and the environment and thus can be used as an effective biological control agent that simultaneously target harmful crop pests and phytopathogens. As a result, the strain is expected to contribute significantly to improving economic efficiency for farmers.

### Brief Description of Drawings

FIG. 1 is a graph comparing the pesticidal effect of spores of *Beauveria bassiana* 331R strain, and the culture and blastospores obtained by culturing the strain in broth on *Tetranychus urticae.*
FIG. 2 is a graph comparing the pesticidal effect of *Beauveria bassiana* 331R spores on *Myzus persicae* and *Aphis gossypii.*
FIG. 3 is a graph comparing the pesticidal effect of cultures obtained by culturing *Beauveria bassiana* 331R strain in broth on *Myzus persicae* and *Aphis gossypii.*
FIG. 4 is a graph comparing the fungicidal activity of cultures obtained by culturing *Beauveria bassiana* 331R strain in broth against *Botrytis cinerea.*
FIG. 5 is a graph showing the fungicidal activity of cultures obtained by culturing *Beauveria bassiana* 331R strain in broth against *Colletotrichum acutatum.*
FIG. 6 is a graph showing the fungicidal activity of cultures obtained by culturing *Beauveria bassiana* 331R strain in broth against *Colletotrichum fructicola.*

### Best Mode for Carrying out the Invention

The present disclosure provides a novel *Beauveria bassiana* 331R strain (Accession No.: KACC83066BP), characterized by concurrent control activity against both plant pests and phytopathogens.

As used herein, the term "control" refers to the ability of a substance to significantly increase the mortality rate of crop pests or inhibit the growth rate of phytopathogens.

Typical obstacles in plant cultivation include various pests and diseases, and to address these issues, pesticides, bactericides, fungicides, and virus control agents are used. In reality, each of these agents must be used to control various pests and diseases, and there are hardly any effective agents capable of controlling them simultaneously.

In pursuit of developing a microbial agent capable of simultaneously controlling both pests and diseases more effectively, the present inventors discovered that the *Beauveria bassiana* strain 331R, isolated during the rearing process of *Tetranychus urticae,* exhibited excellent pesticidal power and high antimicrobial activity against various phytopathogens.

Therefore, the inventors have deposited the identified *Beauveria bassiana* 331R strain with the Korean Agricultural Culture Collection (KACC) on April 15, 2022, under Accession No. KACC83066BP.

Furthermore, the present inventors conducted experiments to verify the pesticidal activity of the *Beauveria bassiana* 331R strain against pests and analyzed the pesticidal activity of the *Beauveria bassiana* strain 331R, its spores, spores obtained from a culture in broth (blastospores), and the culture (culture broth which includes metabolites secreted from the strain with the biomass removed).

The results demonstrated that the conidia, blastospores, and culture medium of the *Beauveria bassiana* 331R strain exhibited high pesticidal activity against *Tetranychus urticae, Myzus persicae,* and *Aphis gossypii.*

Specifically, according to an embodiment of the present disclosure, when a conidia suspension formed in grain medium was sprayed on 30 spider mites (*Tetranychus urticae*) and observed daily for 7 days, the *Beauveria bassiana* 331R strain showed about 99% or more high pesticidal activity after 7 days. The treatment with the blastospore suspension showed about 100% pesticidal activity after 7 days, and the treatment with only the culture medium showed about 89% or more high pesticidal activity after 7 days. Furthermore, simultaneous treatment with spores formed in grain medium or blastospores and culture medium from liquid culture showed about 100% high activity after 7 days, and both cases demonstrated a faster pesticidal effect due to the mixed treatment with the culture medium.

Moreover, an analysis of the pesticidal activity against *Myzus persicae* and *Aphis gossypii* showed that the conidia of the *Beauveria bassiana* 331R strain exhibited nearly 100% high pesticidal activity against both pests, and the culture medium of the *Beauveria bassiana* 331R strain showed about 71.3% pesticidal rate against *Myzus persicae* and about 63.3% against *Aphis gossypii.*

Therefore, the present inventors have determined that the conidia, blastospores, and the culture medium of the *Beauveria bassiana* 331R strain can be usefully employed as control agents for managing various pests.

Additionally, in order to confirm the fungicidal activity of the strain of the present disclosure, analysis was made of biocidal activity against *Colletotrichum fructicola,* which is a pathogen of strawberry anthracnose, *Colletotrichum acutatum,* which is a pathogen of pepper anthracnose, and *Botrytis cinerea,* which is a pathogen of gray mold. The analytical results demonstrated that the strain exhibits excellent control activity against these phytopathogens.

Therefore, the present disclosure provides the *Beauveria bassiana* 331R strain (Accession No.: KACC83066BP), which has concurrent control activity against both pests and phytopathogens.

The pests that can be controlled by the strain according to this disclosure may include, but are not limited to, *Tetranychus urticae, Myzus persicae,* or *Aphis gossypii.*

Moreover, the strain according to this disclosure is characterized by having fungicidal activity against fungi that cause plant diseases.

The types of fungi in the present disclosure may include, but are not limited to, *Botrytis cinerea* T3-4, *Colletotrichum acutatum* 15AS32, or *Colletotrichum fructicola* CGF160401.

Furthermore, the present disclosure may provide a composition comprising the strain of the present disclosure, spores thereof, a culture thereof, a concentrate of the culture, or a dried product of the culture as an active ingredient for the simultaneous control of pests and plant diseases.

The culture can be mass-produced using conventional microbial cultivation methods and may utilize, as a culture medium, a broth containing carbon sources, nitrogen sources, vitamins, and minerals. Possible carbon sources include starch, sucrose, citrate, maltose, glucose, mannitol, glycerol, or xylose.

Additionally, the culture may be used in the form of the culture itself containing the strain, or may be the culture medium after the biomass is removed therefrom. Alternatively, the culture medium may be removed from the culture through centrifugation or filtration processes to recover only the concentrated biomass. These steps may be performed by a skilled person as needed. The culture or concentrated biomass may be preserved without loss of activity by conventional methods such as freezing or lyophilization.

Aiming for stable formulation suitable for practical use, the composition of the present disclosure may additionally include one or more substances such as water, carriers, diluents, surfactants, efficacy enhancers, inorganic salts, adjuvants, binders, and bulking agents, besides the active ingredients mentioned (strain, culture of the strain, culture medium, concentrate of the culture, or dried form of the culture).

The surfactants are understood to be amphiphilic substances containing both hydrophilic and lipophilic groups, characterized by excellent cleansing, dispersing, emulsifying, solubilizing, wetting, sterilizing, foaming, and penetrating properties. In the composition of the present invention, the surfactants act to hydrate, suspend, and disperse the active ingredient (strain or culture thereof) effectively to express the efficacy of the composition.

As the surfactants, anionic surfactants such as sodium or calcium salts of sulfonates, e.g., alkylbenzene sulfonates, alkylnaphthalene sulfonates, lignin sulfonates, alkylnaphthalene sulfonate formalin condensate, polyoxyalkylene alkylphenyl sulfonate, etc., sodium or calcium salts of sulfates, e.g., alkyl sulfate, polyoxyalkylene sulfate, polyoxyalkylene phenylsulfate, etc. and sodium or calcium salts of succincates, such as naphthalene sulfosuccinate, dialkyl sulphosuccinates, polyoxyalkylene succinate, etc., and nonionic surfactants such as ethoxylated alkyl ethers, polyoxyalkylene alkyl phenyl polymers, and polyalcohols may be used alone or in combination. These examples are illustrative, and it is easily understood by those skilled in the art that other surfactants can also be used.

The bulking agents, used in conjunction with surfactants, are substances that can adsorb and granulate the surfactants. Examples include starch, soybean meal, wheat bran, granular cellulose, urea, diatomaceous earth, zeolite, bentonite, talc, kaolin, pyrophyllite, white carbon, etc., which can be used alone or in combination.

The binders are substances used to bind the components within the composition together. Examples include water-soluble starch, dextrin, carboxymethylcellulose, sodium polyacrylate, polyvinyl alcohol, gum arabic, or xanthan gum, which can be used alone or in combination.

Additionally, the present disclosure provides a method for simultaneous control of pests and plant diseases, the method including a step of spraying the composition for simultaneous control of pests and plant diseases of the present disclosure in areas infested with pests and diseases.

The areas where the composition of the present disclosure may be sprayed include soil or crops, and examples of the crops include, but are not limited to, eggplants, peppers, pears, apples, cucumbers, roses, cherries, persimmons, safflowers, plums, pepper trees, tangerines, potatoes, tobacco, cotton, evergreens, spurge, spear trees, goji berries, Zelkova trees, apricot trees, plum trees, or jujube trees.

### Mode for Carrying out the Invention

A better understanding of the present disclosure may be obtained through the following examples which are set forth to illustrate, but are not to be construed to limit, the scope of the present invention.

### <Preparation Examples and Experimental Methods>

### Pest Rearing Conditions

*Tetranychus urticae* were fed kidney beans and reared under laboratory conditions at a temperature of 25°C and a relative humidity of 70%, with a light cycle of 12L:12D, for experimental use.

*Myzus persicae* were fed spring cabbage while *Aphis gossypii* were fed cucumber leaves. They were maintained at a temperature of 25°C, relative humidity of 70%, and a light cycle of 12L:12D, and separated using an art brush for experimental use.

### Solid Culture

Solid culture of the isolated and identified *Beauveria bassiana* 331R strain was conducted as follows: The strain was cultured on potato dextrose agar (PDA) plates at 25°C for 14 days. Thereafter, conidia were scraped from the PDA plates and resuspended in a 0.01% Tween-80 (Difco, USA) solution for collection. The conidial suspension was vigorously stirred and filtered through cheesecloth to remove mycelial debris. A hemocytometer was used to measure the conidia concentration.

For grain medium, millet was placed in a polyethylene bag and half the volume of the grains was filled with water containing 0.15% citric acid. After treatment at 95°C for 15 minutes and autoclaving at 121°C for 30 minutes, the grains were cooled and inoculated with the liquid cultured fungus and then incubated at 25°C for 14 days. Thereafter, the fungus-grown grains were vigorously stirred in a 0.02% Tween-80 solution and the conidia were collected, then the conidia concentration was measured using a hemocytometer.

### Liquid Culture

Conidia suspensions obtained from solid culture were pre-checked for germination in SDAY (Sabouraud dextrose agar with 1% yeast extract, pH 5.6) medium supplemented with 0.05% benomyl (benomyl, 95% active ingredient) in SDAY+B medium, and only those showing a germination rate of more than 90% were used. The conidia suspension was inoculated into PDB (potato dextrose broth, pH 5.6) and cultured at 25°C, 150 rpm, in dark conditions for 14 days. After 14 days of cultivation, the culture product was centrifuged to separate the biomass and culture medium. The obtained culture medium was first filtered through filter paper (ADVANTEC, No. 2) and then adjusted to pH 5.6 for fungicidal activity testing. After pH adjustment, it was secondarily filtered through a 0.45 um filter to obtain a purified culture medium used in the experiments. Blastospores were collected by centrifuging the separated biomass, washing same twice with sterile distilled water to remove the culture medium, restoring the original volume with distilled water, homogenizing to obtain a suspension, and then filtering out the biomass through sterilized gauze to collect only the blastospores for experimental use.

### Fungal Genomic DNA Extraction

The extraction of genomic DNA from fungi was performed with a partial modification of the chemical lysis method. Fungal biomass grown on media was ground using liquid nitrogen and a mortar and pestle. Approximately 1 g of biomass was treated with 400 µL of lysis buffer (0.2 M Tris-HCl pH 7.5, 0.5 M NaCl, 10 mM EDTA pH 8.0, 1% w/v SDS) and an equal volume of phenol-chloroform-isoamyl alcohol (25:24:1). The mixture was vigorously stirred for 5 minutes, then centrifuged at 9,800 x g for 8 minutes. The supernatant was transferred to a new tube, and 1 µL of RNase (20 mg/mL, Sigma) was added and incubated at 37°C for 30 minutes. After the reaction, an equal volume of phenol-chloroform-isoamyl alcohol (25:24:1) was added, centrifuged under the same conditions, and the supernatant was collected. DNA precipitation was performed by adding 2.5 times the volume of 100% cold alcohol to the supernatant, followed by centrifugation at 16,000 x g for 10 minutes at 4°C. The DNA pellet thus obtained was washed with 70% alcohol, dried, and dissolved in 50 µL of sterile water.

### Molecular Biological Identification

For molecular biological identification, fungal genomic DNA was extracted. In this regard, a portion of the fungal biomass grown on PDA for two weeks was processed with a fungal DNA extraction buffer (0.2 M Tris-Cl (pH 7.5), 0.5 M NaCl, 10 mM EDTA (pH 8.0) and 1% (w/v) SDS), followed by centrifugation with phenol-chloroform-isoamyl alcohol (25:24:1) to purify the DNA. The purified DNA solution was precipitated using cold ethanol, centrifuged, and dissolved in sterile distilled water for experimental use.

The extracted fungal DNA was targeted for amplification of the ITS (internal transcribed spacer) region using primers ITS1 (5'-TCCGTAGGTGAACCTGCGG-3')and ITS4 (5'-TCCTCCGCTTATTGATATGC-3') in a PCR amplification. The PCR reaction was performed in a total volume of 20 µL with PCR PreMix (250 mM dNTPs, 10 mM Tris-HCl pH 9.0, 30 mM KCl, 1.5 mM MgCl2, Taq DNA polymerase 1 unit; Bioneer Co., Korea), 1 µL of DNA solution, and 10 pmol of each primer. The reaction conditions included an initial denaturation at 94°C for 5 minutes, followed by 35 cycles of denaturation at 94°C for 30 seconds, annealing at 53°C for 30 seconds, and extension at 72°C for 1 minute, with a final extension at 72°C for 10 minutes.

After the PCR reaction, the amplified products were analyzed by electrophoresis on a 1.0% agarose gel and purified using the Power Gel Extraction Kit (Dyne Bio Inc., Korea). The purified PCR products were sent for direct sequencing to Macrogen (Korea). The sequences were aligned using Multalin (http://multalin.toulouse.inra.fr/multalin/) and compared to other reported fungal sequences using the NCBI BLAST search tool.

### Phytopathogens

The phytopathogenic fungi used for the fungicidal activity experiments included *Botrytis cinerea* T3-4, which is a causal agent of gray mold, *Colletotrichum acutatum* 15AS32, which is a causal agent of pepper anthracnose, and *Colletotrichum fructicola* CGF160401, which is a causal agent of strawberry anthracnose. These pathogenic fungi were obtained from the Plant Mycopathology Laboratory at Chungbuk National University and were inoculated on PDA media. *Botrytis cinerea* was cultured at 22°C, while the other strains were cultured at 25°C for experimental use.

### Bioassay

To verify the pest control efficacy of the novel strain of the present disclosure, bioassay samples were prepared using the *Beauveria bassiana* 331R strain. Conidia formed on grain media, blastospores obtained from liquid culture, and culture medium from which mycelia and blastospores were removed from the culture broth were each used in the bioassay. A suspension of spores with a viability exceeding 90% were used, and their viability was determined on SDAY+B media.

### ① Pesticidal Activity Assay

The bioassay for *Tetranychus urticae, Myzus persicae,* and *Aphis gossypii* involved placing a 25 mm diameter host plant leaf in a 60 mm petri dish containing moistened filter paper. Thirty pests were placed on the leaf, and then 1 mL of conidia suspension (1x10⁸ conidia/mL) collected from grain media, 1 mL of blastospores (1x10⁸ blastospore/mL) collected from liquid culture, and 1 mL of the culture medium were sprayed. The dishes were maintained at 25°C with a 12L:12D light cycle and 70% humidity, observed at 24-hour intervals daily, with a control group treated only with 0.02% Tween-80 solution. The bioassay was conducted in 3 replicate.

### ② Fungicidal Activity of Fungal Culture Broth

Fungicidal activity testing of the fungal culture broth was conducted as follows: *Botrytis cinerea* was cultured on PDA media to induce spore formation. Spores scraped from the fungal surface were suspended in 10% PDB medium. The collected spore suspension was filtered through sterile gauze to remove B. cinerea mycelia, and spore count was determined using a hemocytometer. Approximately 2x10⁴ spores/mL were adjusted using 2X PDB medium (pH 5.6), inoculated in an amount of 100 µL into a 96-well plate. After inoculation, the culture medium of the entomopathogenic fungus *Beauveria bassiana* 331R was diluted with sterile water to make 1% (v/v) and 10% (v/v) solutions, which were then inoculated at 100 µL each. These were incubated at 22°C in dark conditions for 48 hours, after which the absorbance at 595 nm was read to observe the growth of *Botrytis cinerea.* The control group used 100 µL of sterile water instead of culture medium. The same method was applied to assess the fungicidal activity against *Colletotrichum acutatum* 15AS32 and *Colletotrichum fructicola* CGF160401.

### Statistical Analysis

Pesticidal and fungicidal activity assessments were performed in 3 replicate, and the results were analyzed using the SPSS statistical software with a significance level set at p < 0.05. Data were presented as mean ± standard error (SE).

### EXAMPLE 1

### Selection of Pathogenic Strain with Control Activity Against Pests and Phytopathogens

Mycosis was observed in *Tetranychus urticae* being reared indoors, from which fungi were isolated. The fungal spores covering the dead *Tetranychus urticae* were separated and inoculated on a selective medium for entomopathogenic fungi, SDAY+B, and cultured at 25°C in dark conditions for 7 days. The fungi proliferated on the selective medium were then inoculated onto PDA media and further cultured at 25°C in dark conditions for 14 days.

To molecular-biologically identify the isolated fungus, fungal biomass was collected, and DNA was extracted. The ITS region was amplified by PCR, and its sequence was determined. Comparative analysis of the determined sequence with previously reported strains revealed that it was a strain of *Beauveria bassiana,* which was confirmed to be a novel strain not previously known. Consequently, the inventors named this newly isolated strain *"Beauveria bassiana* 331R" and deposited it with the Korean Agricultural Culture Collection (KACC) on April 15, 2022, under accession no. KACC83066BP. Additionally, the DNA sequence of the ITS region for the identified *Beauveria bassiana* 331R strain is as follows:

### < ITS Region DNA Sequence of Beauveria bassiana 331R Strain>

CCCTTCTGTG AACCTACCTA TCGTTGCTTC GGCGGACTCG
CCCCAGCCCG GACGCGGACT
GGACCAGCGG CCCGCCGGGG ACCTCAAACT CTTGTATTCC
AGCATCTTCT GAATACGCCG
CAAGGCAAAA CAAATGAATC AAAACTTTCA ACAACGGATC
TCTTGGCTCT GGCATCGATG
AAGAACGCAG CGAAATGCGA TAAGTAATGT GAATTGCAGA
ATCCAGTGAA TCATCGAATC
TTTGAACGCA CATTGCGCCC GCCAGCATTC TGGCGGGCAT
GCCTGTTCGA GCGTCATTTC
AACCCTCGAC CTCCCCTTGG GGAGGTCGGC GTTGGGGACC
GGCAGCACAC CGCCGGCCCT
GAAATGGAGT GGCGGCCCGT CCGCGGCGAC CTCTGCGTAG
TAATACAGCT CGCACCGGAA
CCCCGACGCG GCCACGCCGT AAAACACCCA ACTTCTGAAC
GTTGACCTCG AATCAGGTAG
GAC

### EXAMPLE 2

### Evaluation of the Pesticidal Activity of Beauveria bassiana 331R Strain Against Pests

The inventors analyzed the pesticidal effects of the *Beauveria bassiana* 331R strain, isolated and identified herein, using the following methods:

### <2-1> Pesticidal Activity Against Tetranychus urticae

The pesticidal activity against *Tetranychus urticae* was evaluated using conidia produced on grain media, and the products of liquid culture, which included blastospores and the culture medium with removed fungal biomass.

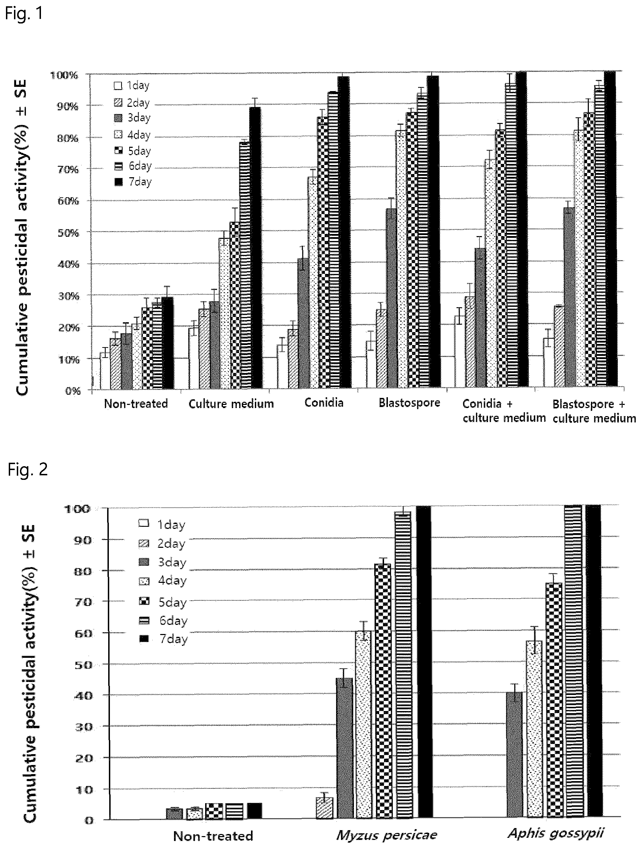

The results showed that the culture broth alone, after the removal of biomass, exhibited a pesticidal rate of about 89% after 7 days. The groups treated solely with spores produced on grain media or blastospores obtained from liquid culture showed a high pesticidal rate of about 99%. The groups that received a mixed treatment of culture medium with either conidia or blastospores showed almost 100% pesticidal rate (FIG. 1). Moreover, blastospores produced in liquid culture showed a faster and higher pesticidal rate from day 3 compared to those produced on grain media. The groups with a mixed treatment of conidia or blastospores and culture medium showed a faster and higher pesticidal rate from day 3 compared to the groups treated singly.

These results demonstrate that the novel *Beauveria bassiana* 331R strain isolated and identified in the present disclosure exhibits excellent pesticidal effects against *Tetranychus urticae,* whether using conidia, blastospores, or culture medium.

### <2-2> Pesticidal Activity Against Aphids

Furthermore, in order to confirm the pesticidal activity of the *Beauveria bassiana* 331R strain against aphids, the inventors evaluated the pesticidal effects of the *Beauveria bassiana* 331R strain's conidia and culture medium against *Myzus persicae* and *Aphis gossypii.*

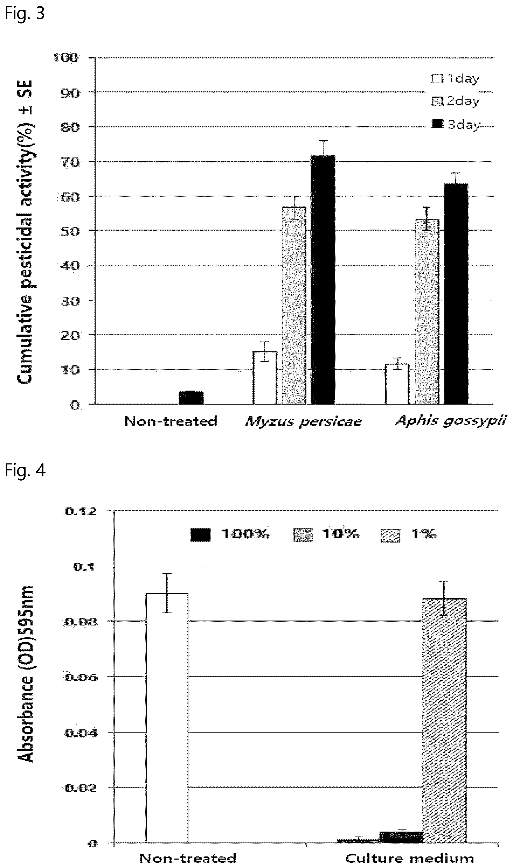

The results showed that treatment with spores formed on grain media resulted in a pesticidal rate of over 40% against both types of aphids after 3 days, and nearly 100% after 7 days (FIG. 2). Additionally, the groups treated with the culture broth obtained after removing the biomass exhibited a pesticidal rate of over 10% after just 1 day. By day 3, the treatment showed about 71.3% efficacy against *Myzus persicae* and about 63.3% against *Aphis gossypii* (FIG. 3).

These findings confirm that the *Beauveria bassiana* 331R strain, in addition to its effects on *Tetranychus urticae,* also exhibits high pesticidal efficacy against aphids that are harmful to plants and crops.

### EXAMPLE 3

### Evaluation of Fungicidal Activity of Beauveria bassiana 331R Strain Against Phytopathogens

In order to confirm the fungicidal activity of the *Beauveria bassiana* 331R strain against phytopathogens, the inventors further evaluated the fungicidal activity using the culture medium previously utilized in Example 2.

Specifically, to assess the fungicidal activity of the *Beauveria bassiana* 331R culture medium against *B. cinerea,* the strain was cultured in PDB broth (pH 5.6) for two weeks. After culturing, the biomass was removed from the culture product, and the remaining culture medium was adjusted to pH 5.6 ± 0.2. The purified culture medium was then assayed for fungicidal activity against *B. cinerea.* The results showed very high fungicidal activity both at 100% (v/v) concentration and when diluted to 10% (v/v) concentration (see FIG. 4).

Additional assay was made for fungicidal activity against other phytopathogenic fungi. In this regard, the same method was employed to analyze the fungicidal activity against *Colletotrichum acutatum* 15AS32 and *Colletotrichum fructicola* CGF160401.

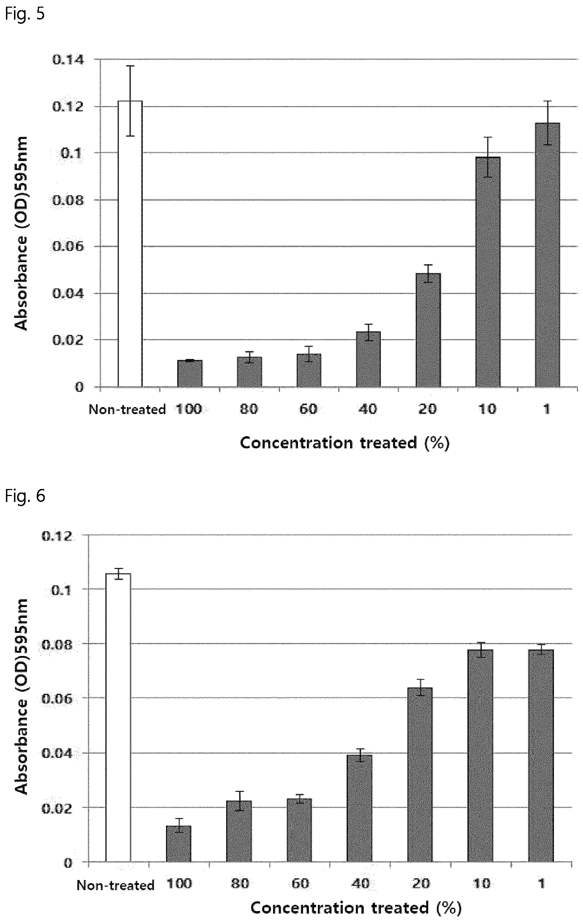

The results indicated that the culture medium derived from the *Beauveria bassiana* 331R strain exhibited observable fungicidal activity against both *Colletotrichum acutatum* 15AS32 and *Colletotrichum fructicola* CGF160401. The fungicidal activity was dependent on the concentration of the culture medium applied. Specifically, up to a 40% (v/v) concentration of the culture medium showed relatively high fungicidal activity against both pathogens (see FIGS. 5 and 6).

Through these findings, the present inventors confirmed that the *Beauveria bassiana* 331R strain exhibits high fungicidal activity against ***B.** cinerea,* pepper anthracnose fungus *Colletotrichum acutatum* 15AS32, and strawberry anthracnose fungus *Colletotrichum fructicola* CGF160401.

The cumulative results demonstrate that the *Beauveria bassiana* 331R strain according to the present disclosure exhibits excellent pesticidal properties against pests such as *Myzus persicae, Aphis gossypii,* and *Tetranychus urticae,* utilizing all forms of its cultured products, including spores. Additionally, it also shows high fungicidal activity against phytopathogenic fungi such as ***B.** cinerea, Colletotrichum acutatum* 15AS32, and *Colletotrichum fructicola* CGF160401. This confirms that the 331R strain is a valuable organism capable of effectively controlling both pests and phytopathogens simultaneously.

While the present disclosure has been particularly shown and described with reference to exemplary embodiments thereof, it will be understood by those of ordinary skill in the art that various changes in form and details may be made therein without departing from the spirit and scope of the present invention as defined by the following claims. The exemplary embodiments should be considered in descriptive sense only and not for purposes of limitation. Therefore, the scope of the invention is defined not by the detailed description but by the appended claims, and all differences within the scope will be construed as being included in the present invention.

Depositary Authority: Korean Agricultural Culture Collection (KACC)
Accession Number: KACC83066BP
Deposit Date: April 15, 2022

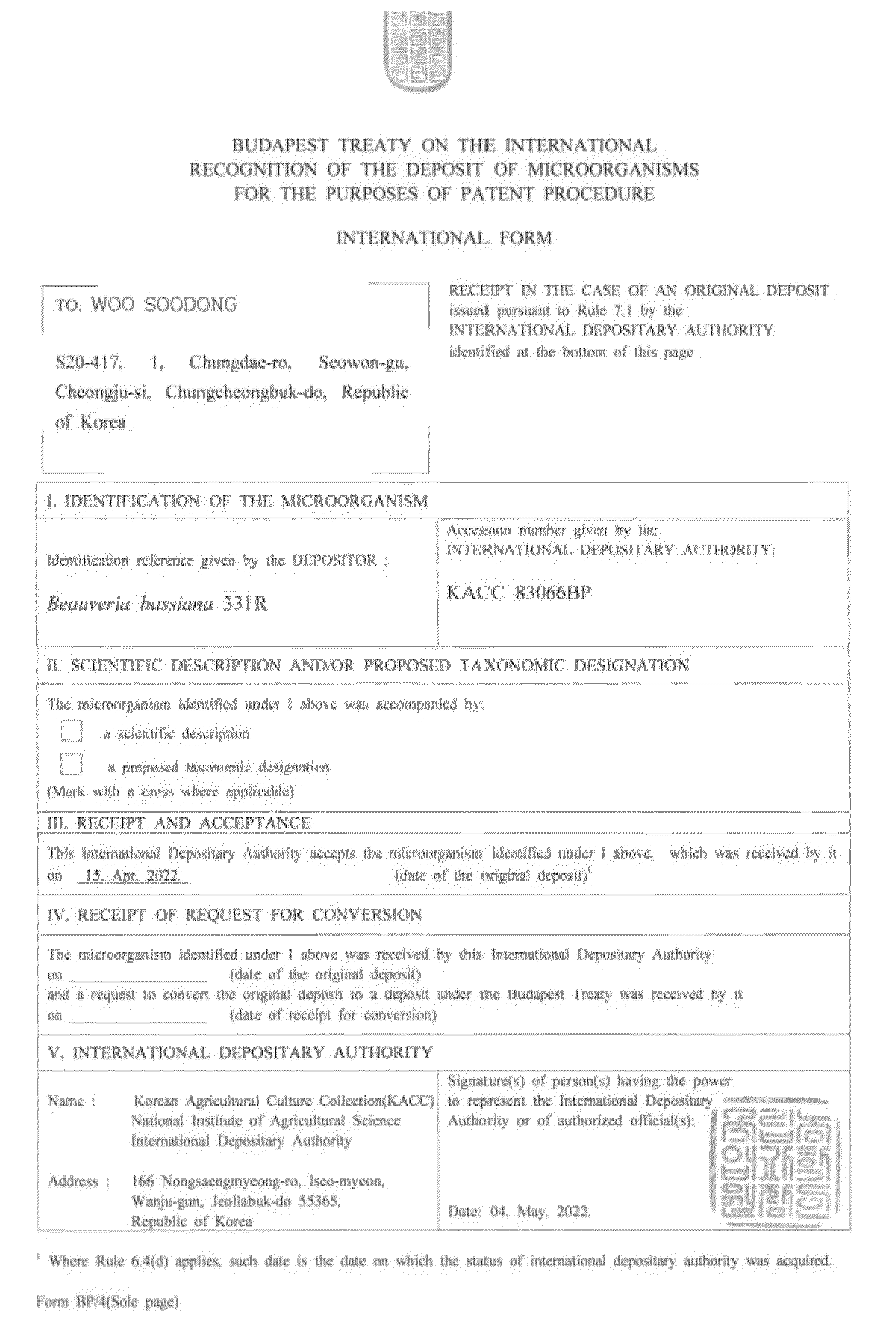

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO. WOO SOODONG

S20-417, 1, Chungdae-ro, Seowon-gu,
Cheongju-si, Chungcheongbuk-do, Republic
of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the DEPOSITOR :
Beauveria bassiana 331R | Accession number given by the
INTERNATIONAL DEPOSITARY AUTHORITY:
KACC 83066BP
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which was received by it
on 15. Apr. 2022. (date of the original deposit)¹
IV. RECEIPT OF REQUEST FOR CONVERSION
The microorganism identified under I above was received by this International Depositary Authority
on ________________ (date of the original deposit)
and a request to convert the original deposit to a deposit under the Budapest Treaty was received by it
on ________________ (date of receipt for conversion)
V. INTERNATIONAL DEPOSITARY AUTHORITY
Name : Korean Agricultural Culture Collection(KACC)
National Institute of Agricultural Science
International Depositary Authority

Address : 166 Nongsaengmyeong-ro, Iseo-myeon,
Wanju-gun, Jeollabuk-do 55365,
Republic of Korea | Signature(s) of person(s) having the power
to represent the International Depositary
Authority or of authorized official(s):

Date: 04. May. 2022.

¹ Where Rule 6.4(d) applies, such date is the date on which the status of international depositary authority was acquired.

Form BP/4(Sole page)

## Claims

1. A *Beauveria bassiana* 331R strain (Accession Number: KACC83066BP), exhibiting simultaneous control activity against both pests and phytopathogens.

2. The *Beauveria bassiana* 331R strain (Accession Number: KACC83066BP) of Claim 1, wherein the pests are *Tetranychus urticae, Myzus persicae,* or *Aphis gossypii.*

3. The *Beauveria bassiana* 331R strain (Accession Number: KACC83066BP) of Claim 1, wherein the control activity against phytopathogens is fungicidal activity against fungi causing plant diseases.

4. The *Beauveria bassiana* 331R strain (Accession Number: KACC83066BP) of Claim 3, wherein the fungi are *Botrytis cinerea* T3-4, *Colletotrichum acutatum* 15AS32, or *Colletotrichum fructicola* CGF160401.

5. A composition for simultaneous control of pests and plant diseases, comprising the *Beauveria bassiana* 331R strain of Claim 1, spores thereof, a culture thereof, a concentrate of the culture, or a dried product of the culture as an active ingredient.

6. The composition of Claim 5, wherein the pests are *Tetranychus urticae, Myzus persicae,* or *Aphis gossypii.*

7. The composition of Claim 5, wherein the plant diseases are caused by *Botrytis cinerea* T3-4, *Colletotrichum acutatum* 15AS32, or *Colletotrichum fructicola* CGF160401.

8. A method for simultaneous control of pests and plant diseases, the method comprising a step of spraying the composition of Claim 5 in areas infested with pests and diseases.

9. The method of Claim 8, wherein the area is soil or crops.

10. The method of Claim 9, wherein the crops are eggplants, peppers, tomatoes, strawberries, cabbages, pears, apples, cucumbers, roses, cherries, persimmons, safflowers, plums, pepper trees, tangerines, potatoes, sweet potatoes, tobacco, cotton, evergreens, spurge, spear trees, goji berries, Zelkova trees, apricot trees, plum trees, or jujube trees.
